# EUROPÄISCHE PATENTANMELDUNG

(11) **EP 2 826 853 A1**
(43) Veröffentlichungstag der Anmeldung: **21.01.2015**
(21) Anmeldenummer: 14177428.1
(22) Anmeldetag: 17.07.2014
(51) Int. Cl.: C12M 1/107, C12M 1/00, C12M 1/34, C12M 1/36

(54) **Biogas-Anlage sowie Verfahren zu ihrem Betrieb**

(30) Priorität: 19.07.2013 DE 102013107754
(71) Anmelder: Lutz, Peter, 81925 Unterföhring (DE)
(72) Erfinder: Lutz, Peter, 81925 Unterföhring (DE)
(74) Vertreter: Hansmann & Vogeser

(57) **Zusammenfassung**

Um in den Fermentern **(1)** anfallendes Schwachgas dennoch im Biogasverbraucher **(10)** nutzen zu können, wird erfindungsgemäß vorgeschlagen, dieses Schwachgas in geringer Menge dem anfallenden Biogas zuzuführen, so dass das entstehende Gasgemisch immer noch für den Biogas-Verbraucher **(10)** nutzbar ist.

## Beschreibung

### I. Anwendungsgebiet

Die Erfindung betrifft eine Biogas-Anlage sowie ein Verfahren zu ihrem Betrieb.

### II. Technischer Hintergrund

Heute wird Biomasse, beispielsweise Silage oder Bio-Abfälle oder die organische Fraktion des Hausmülls, auf zwei grundsätzlich unterschiedliche Arten anaerob vergoren:
- einerseits im Nassgär-Verfahren, bei dem die Biomasse in Form einer pumpfähigen Schlempe vorliegt,
- andererseits im hier vorliegenden Feststoff-Verfahren, bei der die Biomasse, nur durch das Animpfen mit bereits vergorenem Material, in die Fermenter eingebracht wird.

Dabei sammelt sich das entstehende, brennbare Biogas im Freiraum oberhalb der Biomasse in jedem Fermenter und kann von dort aus abgesaugt und einem Verbraucher zugeführt werden.

In diesem Zusammenhang ist der luft-und gasdichte Verschluss der Fermenter sehr wichtig, denn falls in den Fermenter von außen Luft und damit Sauerstoff eindringen würde, kann im Inneren des Fermenters ein explosionsfähiges Gas-Gemisch entstehen.

Um dies zu verhindern, wird im Gasvolumen jedes Fermenters ein leichter Überdruck, zum Beispiel zwischen **5** mbar und **30** mbar, gehalten, um auf diese Art und Weise bereits das Eindringen von Luftsauerstoff von außerhalb des Fermenters zu verhindern. In der Biogas-Sammelleitung ist zwar ein Verdichter angeordnet, der das Biogas absaugt und dem Biogas-Verbraucher zufördert, aber auch dieser Verdichter ist so gesteuert, dass der besagte Überdruck in den einzelnen Fermentern erhalten bleibt.

Dennoch wird die Gaszusammensetzung im Fermenter ständig kontrolliert, beispielsweise hinsichtlich des Sauerstoffanteils oder des Kohlendioxid-Anteils, um bei entstehendem explosivem Gas-Gemisch Gegenmaßnahmen ergreifen zu können.

Eine Situation, in der immer Luftsauerstoff in den Fermenter eindringen und sich mit dort vorhandenem Biogas zu einem explosionsfähigen Gemisch verbinden kann, ist die Öffnung der Schließvorrichtung eines Fermenters nach beendetem Gärvorgang, um die vergorene Biomasse aus dem Fermenter zu entnehmen.

Auch hier muss vor dem Öffnen eine Gegenmaßnahme durchgeführt werden, um die Bildung eines explosionsfähigen Gemisches zu vermeiden.

Eine solche Gegenmaßnahme ist das Einpressen von Inertgas in den entsprechenden Fermenter, das so genannte Spülen. Inertgas in diesem Zusammenhang ist ein unbrennbares Gas, welches auch zusammen mit dem Biogas kein explosionsfähiges Gemisch ergeben kann, beispielsweise das Abgas des Biogas-Verbrauchers, welcher in der Regel ein Gasmotor ist.

Dadurch wird das darin befindliche Biogas gemischt mit dem Abgas zu sogenanntem Schwachgas und aus dem Fermenter herausgedrückt und entweder über eine Fackel abgefackelt oder auf andere Art und Weise verarbeitet, da insbesondere Schwachgas zu wenig brennbare Anteile enthält, um in dem Biogas-Verbraucher dieser Biogasanlage, insbesondere einem Gasmotor, benutzt werden zu können.

Diese Lösung besitzt jedoch den Nachteil, dass das aus dem Fermenter herausgepresste Gasgemisch, das sogenannte Schwachgas, für die Nutzung im Biogas-Verbraucher verloren ist und somit die Effizienz der Biogas-Anlage senkt. Eine Zuführung des Schwachgases alleine zum Biogas-Verbraucher ist jedoch wegen der sich dann zu schnell ändernden Qualität des Gasgemisches aufgrund des zugemischten Abgases nicht möglich.

Unter Schwachgas wird in diesem Zusammenhang jedes Gas-Gemisch verstanden, welches Methan enthält, insbesondere noch so viel Methan enthält, dass es brennbar ist, aber dennoch nicht dem Biogas-Verbraucher zugeführt werden darf, beispielsweise weil es explosionsgefährdet ist oder weil sein Anteil an Methan zu gering ist für das Verwerten im Biogas-Verbraucher.

### III. Darstellung der Erfindung

### a) Technische Aufgabe

Es ist daher die Aufgabe gemäß der Erfindung, eine Biogasanlage und ein Verfahren zu ihrem Betrieb zur Verfügung zu stellen, mit dessen Hilfe das bei der Spülung des Gasraumes eines Fermenters entstehende Schwachgas im Biogas-Verbraucher genutzt werden kann.

### b) Lösung der Aufgabe

Diese Aufgabe wird durch die Merkmale der Ansprüche **1** und **8** gelöst. Vorteilhafte Ausführungsformen ergeben sich aus den Unteransprüchen.

Der **Grundgedanke** der Erfindung besteht darin, anfallendes Schwachgas unter Ausnutzung der darin enthaltenen Energie im Biogas-Verbraucher zu verwerten, und nicht einfach abzufackeln oder ohne Verbrennung in die Umgebung zu entlassen.

Dabei wird davon ausgegangen, dass bei einer regulär betriebenen Biogas-Anlage die Menge an anfallendem Schwachgas im Vergleich zur anfallenden Menge an Biogas relativ gering ist, das Schwachgas nur über jeweils relativ kurze Zeitintervalle anfällt und darüber hinaus das anfallende Biogas deutlich mehr Methan enthält, als es minimal für die Verwendung im angeschlossenen Biogas-Verbraucher erforderlich wäre.

Unter diesen Voraussetzungen ist es möglich, das Schwachgas, selbst wenn es einen Anteil an Sauerstoff aufweist, dem Biogas vor dessen Verwertung im Biogas-Verbraucher zuzuführen, jedoch maximal in so geringer Menge, dass das entstehende Gasgemisch zum einen nicht mehr explosionsgefähr-det ist und zum anderen immer noch genug Methan aufweist, um problemlos im Biogas-Verbraucher und/oder einem alternativ oder zusätzlich vorhandenen Inertgaserzeuger, z.B. einem Heizkessel, verwendet zu werden.

Die Qualität, hinsichtlich des prozentualen Methangehaltes, des dem Biogas-Verbraucher zugeführten Gemisches aus Biogas und Schwachgas wird dadurch zwar reduziert, die zugeführte absolute Methan-Menge jedoch erhöht, so dass insgesamt eine höhere Energie-Effizienz der Biogas-Anlage erreicht wird.

Das in einem Fermenter entstehende Schwachgas kann in einem Schwachgas-Zwischenspeicher gepuffert werden, um es später dem Biogas zuzumischen, oder es kann sofort entweder einem oder mehreren der übrigen Fermentern zugeführt werden, in dessen Gasraum es sich mit dem dort entstehenden Biogas vermischt, oder der Biogas-Sammelleitung zugeführt werden. Hinsichtlich der **Vorgehensweise** ist eine zentrale oder eine dezentrale Zudosierung des Schwachgases möglich:

Bei der zentralen Zudosierung wird das Schwachgas dem Biogas erst in der Sammelleitung für Biogas, also meist erst unmittelbar vor der Gasreinigungsstufe und dem Einbringen in den Biogas-Verbraucher, zugesetzt, natürlich gesteuert mittels einer Dosiersteuerung, damit das entstehende Gasgemisch für den Biogasverbraucher verarbeitbar und dennoch nicht explosionsgefährdet ist.

Diese Lösung ist mit einem geringen Investitionsaufwand verbunden, da nur eine einzige Dosiersteuerung benötigt wird, und auch nur eine einzige Leitung zu der einzigen Einspeisestelle in die Biogas-Sammelleitung erforderlich ist.

Die Erzeugung individueller Gas-Gemische an den Biogas-Entnahmeöffnungen der einzelnen Fermenter ist dadurch jedoch nicht möglich.

Bei der dezentralen Zudosierung wird nach jedem Fermenter separat über eine Dosiersteuerung dessen Biogas die richtige Menge an Schwachgas zugeführt und dadurch gibt jeder einzelne Fermenter separat ein Gasgemisch an die Biogas-Sammelleitung ab, welches für den Biogas-Verbraucher nutzbar ist.

Für die Stelle der dezentralen Zudosierung stehen mindestens zwei Varianten zur Verfügung:

Bei einer Variante wird das Schwachgas direkt an der Biogas-Entnahmeöffnung, vorzugsweise auf deren Außenseite des Fermenters, zugeführt und die entstehende Mischung unmittelbar hinter der Zuführung hinsichtlich bestimmter Parameter, zum Beispiel des Methangehaltes und/oder des Sauerstoffgehaltes, analysiert und abhängig davon der Weiterleitungsweg festgelegt. Dabei wird ein sich abhängig von dem Mischungsverhältnis sehr schnell änderndes Gasgemisch erzeugt.

Bei einer anderen Variante wird das Schwachgas in den Fermenter eingebracht an einem möglichst weit von der Biogas-Entnahmeöffnung entfernten Punkt und durchläuft den Fermenter und vermischt sich auf dem Weg zur Biogas-Entnahmeöffnung mit dem sich im Fermenter bildenden oder bereits vorhandenen Biogas. Auf diese Art und Weise ändert sich die Zusammensetzung des Gasgemisches, welches an der Biogas-Entnahmeöffnung anfällt, nur relativ langsam.

Bei dieser Lösung kann bei dem Fermenter, der eventuell momentan noch gespült wird, und aus welchem das Schwachgas teilweise stammt, je nach Zusammensetzung des aus diesem Fermenter austretenden Schwachgases auf die Zumischung weiteren Schwachgases, insbesondere aus einem Schwachgas-Zwischenspeicher, welches ja eine andere Zusammensetzung haben kann, verzichtet werden.

Der Vorteil dieser Lösung besteht darin, dass sichergestellt ist, dass bei jedem einzelnen Fermenter ab der Stelle der Zumischung des Schwachgases kein explosionsgefährdetes Gasgemisch mehr vorliegt, was unter Umständen für die zentrale Zudosierung für den Bereich der Biogas-Sammelleitung bis zur Stelle der Zumischung des Schwachgases nicht sichergestellt werden kann.

Die gemäß der Erfindung typischen **Abläufe** nach dem Befüllen eines Fermenters oder vor dem Entnehmen der Gärreste aus einem Fermenter sind damit folgende:
Nach dem Befüllen des Fermenters:
   - Schließen des Fermenters,
   - Spülung des Fermenters mit Inertgas bis ein bestimmter, insbesondere minimaler, Sauerstoffgehalt und/oder bestimmter, insbesondere maximaler Kohlendioxidgehalt im Fermenter erreicht ist,
   - Schließen der Inertgas-Zufuhr, insbesondere Abgas-Zufuhr in den Fermenter,
   - Einleiten des Schwachgases aus dem Fermenter ganz oder teilweise in den Schwachgas-Speicher oder Zumischung zu dem Biogas aus einem oder mehreren der anderen Fermenter bis ein erster Wert der Methan-Konzentration am Ausgang des Fermenters bzw. des Schwachgasspeichers erreicht ist,
   - Schließen der Schwachgas-Entnahmeöffnung, wenn ein zweiter Wert der Methan-Konzentration am Ausgang des Fermenters erreicht ist,
   - Öffnen der Biogas-Entnahmeöffnung des Fermenters,
   - Zudosierung des Schwachgases aus dem Schwachgasspeicher oder aus einem der Schwachgas produzierenden Fermenter in die Biogas-Sammelleitung oder in einen oder mehrere Fermenter in der Menge, dass das dort entstehende Gasgemisch für den Biogas-Verbraucher nutzbar ist.
Vor dem Öffnen des Fermenters:
   - Schließen der Biogas-Entnahmeöffnung,
   - Öffnen der Schwachgas-Entnahmeöffnung,
   - Zufuhr von Inertgas in den Fermenter,
   - Einleiten des Schwachgases aus diesem Fermenter bis der Methangehalt einen ersten Wert, insbesondere einen bestimmten Mindestwert, oder der Kohlendioxidgehalt einen ersten Wert, insbesondere Höchstwert, erreicht ganz oder teilweise in den Schwachgasspeicher oder Zumischung zu dem Biogas aus einem oder mehreren der anderen Fermenter,
   - Öffnen der Verbindung vom Fermenter zur Fackel und insbesondere Schließen der Schwachgas-Entnahmeöffnung und, wenn der Methangehalt einen zweiten, insbesondere unter dem ersten Wert liegenden, Wert erreicht, oder der Kohlendioxidgehalt des Schwachgases einen zweiten, insbesondere über dem ersten Wert liegenden, Wert erreicht, oder der Verbindung vom Fermenter zum Abluft-Kamin, wenn der Methangehalt einen dritten, insbesondere unter dem zweiten Wert liegenden, Wert erreicht, oder der Kohlendioxidgehalt des Schwachgases einen dritten, insbesondere über dem zweiten Wert liegenden, Wert erreicht,
   - Beenden der Inertgas-Zufuhr, insbesondere der Abgas-Zufuhr und insbesondere Schließen der Schwachgas-Entnahmeöffnung,
   - Öffnen der Abluft-Entnahmeöffnung und Spülen des Fermenters mit Luft, insbesondere über die Frischluft-Zufuhröffnung.

Eine entsprechende **Biogas-Anlage** benötigt zunächst einmal eine Schwachgas-Sammelleitung, die mit jedem Fermenter verbunden ist.

Häufig wird das gesammelte Schwachgas nicht sofort und in der anfallenden Menge dem entstehenden Biogas zugesetzt, sondern es wird zumindest teilweise in einem Zwischenspeicher für Schwachgas gesammelt, beispielsweise einem Foliensack mit entsprechend großem Volumen, aus dem es dann zeitversetzt dem Biogas zugesetzt werden kann.

Ferner ist eine Dosiersteuerung für die Zudosierung von Schwachgas in das Biogas notwendig.

Diese misst beispielsweise den Methangehalt und/oder den Kohlendioxidgehalt und/oder den Sauerstoffgehalt insbesondere vor und/oder hinter der Zuführung des Schwachgases und regelt in Abhängigkeit davon die eingespeiste Menge an Schwachgas in das Biogas, sodass vorgegebene Grenzwerte für die gemessenen Parameter bei dem entstehenden Gasgemisch eingehalten werden.

Ferner sollte jeder Fermenter an der Biogas-Entnahmeöffnung eine Wählsteuerung aufweisen, um das Biogas - oder bei dezentraler Zudosierung von Schwachgas das daraus gebildete Gasgemisch - wahlweise der Biogas-Sammelleitung, der Schwachgas-Sammelleitung, einer Abluft-Sammelleitung oder einer Abfackel-Sammelleitung zuzuführen.

Die Möglichkeit, Gas aus einem Fermenter Abzufackeln oder der Abluft direkt zuzuführen, muss nach wie vor gegeben sein für Notfallsituationen, beispielsweise wenn der einzige vorhandene Biogasverbraucher ausfällt, um das dennoch anfallende Biogas abzufackeln. Eine andere Situation ist der Fall, dass ein Spülvorgang eines Fermenters bereits soweit abgeschlossen ist, dass das austretende Gas keinen nennenswerten Methan-Anteil mehr enthält und zum Beispiel nur noch aus dem Spülgas besteht oder aus der zum Nachspülen verwendeten Luft, und deshalb nicht mehr als Schwachgas für die Rezirkulation verwendet werden kann.

Abhängig davon, ob die Zudosierung von Schwachgas zentral oder dezentral erfolgen soll, muss die Dosiersteuerung dezentral an den einzelnen Stellen der Zudosierung erfolgen, oder eben nur an der einzigen Zuführungsstelle von Schwachgas in das Biogas kurz vor der Zuführung in den Biogas-Verbraucher.

### c) Ausführungsbeispiele

Ausführungsformen gemäß der Erfindung sind im Folgenden beispielhaft näher beschrieben. Es zeigen:
- Fig. **1:**: die Biogas-Anlage in einer ersten Ausführungsform,
- Fig. **2:**: die Biogas-Anlage in einer zweiten Ausführungsform,
- Fig. **3:**: einen einzelnen Fermenter.

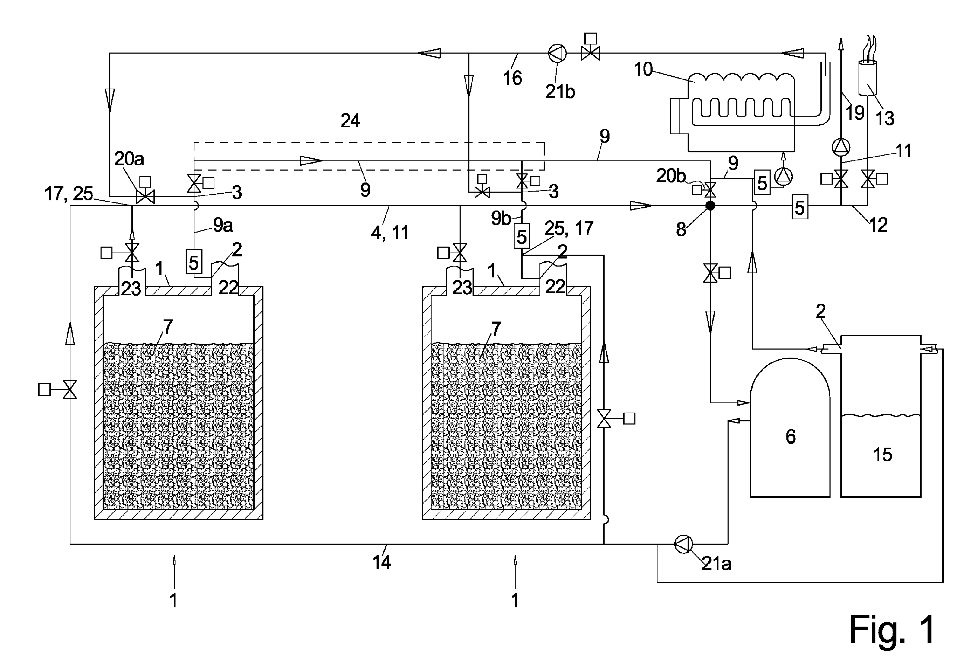

**Figur 1** zeigt eine Biogas-Anlage, bei der nur zwei Fermenter **1** dargestellt sind, was in der Praxis eine unrealistisch geringe Anzahl ist, jedoch für die Prinzipdarstellung ausreicht.

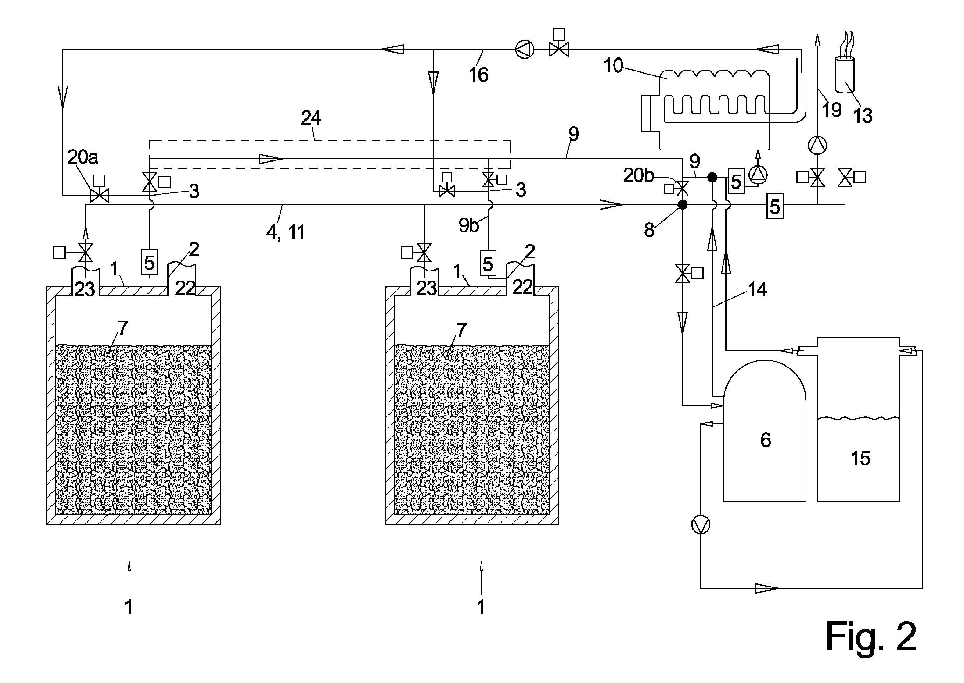

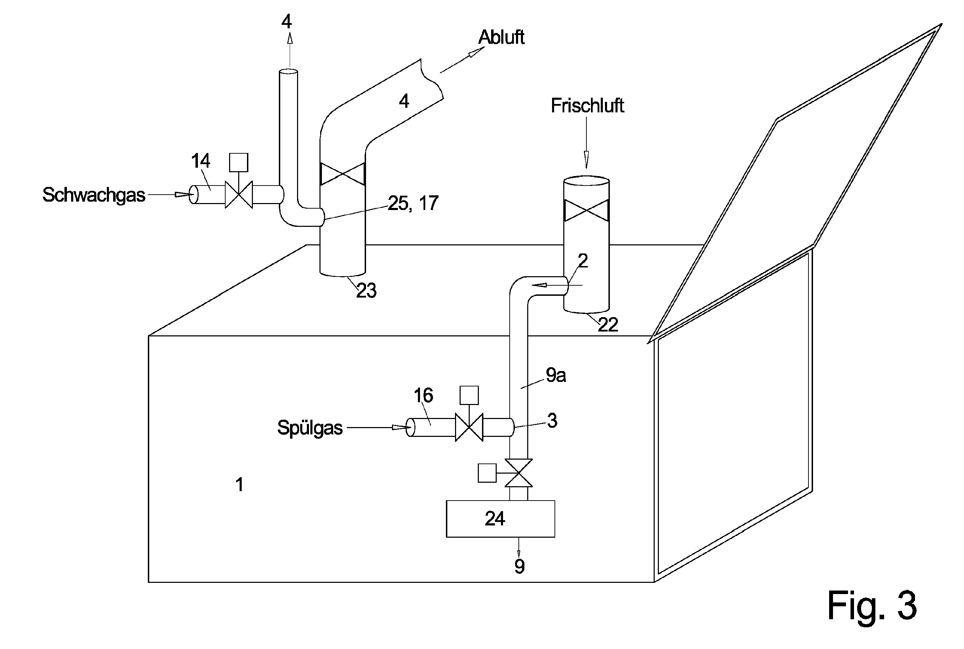

Die Fermenter **1** sind großenteils mit Biomasse **7** gefüllt, und besitzen in ihrer Decke zumindest eine Frischluft-Zufuhröffnung **22** und eine Abluft-Entnahmeöffnung **23.** Diese dienen primär dem Spülen des Fermenters **1** mit Frischluft, wofür ein großer Leitungsquerschnitt benötigt wird, und diese beiden Öffnungen befinden sich - wie die realistische Darstellung eines Fermenters in **Figur 3** zeigt - diagonal gegenüberliegend im Fermenter mit einem möglichst großen Abstand zueinander. Wie die Figuren **1** und **2** zeigen, und nachfolgend im Detail erläutert wird, werden über diese beiden Öffnungen jedoch auch andere gasförmige Komponenten zu- oder abgeführt.

Von der jeweiligen Öffnung aus wird das aus dem Fermenter bei **2** abgesaugte Biogas oder Gasgemisch
- entweder einer Biogas-Sammelleitung **9** zugeführt, die das gesammelte Biogas dem Biogas-Verbraucher **10,** in der Regel einem Gasmotor, als Brennstoff zuführt; der gestrichelte eingefasst die Bereich der Biogas-Sammelleitung ist die Überdrucksicherung **24,** die verhindert, dass selbst bei Ausfall der kompletten Anlage in irgendeinem Bereich ein zu hoher Überdruck an Biogas entstehen kann,
- oder einer Abluft-Sammelleitung **11** zugeführt, die das Biogas über einen Abluftkamin **19** in die Umgebung entlässt,
- oder einer Abfackel-Sammelleitung **12** zugeführt, die das in diesem Fall brennbare Gas einer Fackel **13** zum Abfackeln zuführt,
- oder einer Schwachgas-Sammelleitung **4** zugeführt - die in Figur **1** und **2** identisch mit der Abluft-Sammelleitung **11** ist - die das darin gesammelte Gas einem Zwischenspeicher **6** für Schwachgas zuführt. Dieser kann ein großer Foliensack sein, ein fester Behälter mit offener Oberseite, die von einer, insbesondere dehnbaren, Folie dicht abgedeckt ist oder auch auf andere Art und Weise beschaffen sein.

In jeder der Stichleitungen **9a** und **9b** von der Biogas-Entnahmeöffnung **2,** die sich insbesondere seitlich im Frischluft-Zufuhrstutzen **22** befindet, befindet sich eine Spülgas-Zufuhröffnung **3,** die aus der Spülgas-Versorgungsleitung **16** mit Spülgas, nämlich dem Abgas des Biogas-Verbrauchers **10,** gespeist wird. Der Teil der Stichleitung **9a** oder **9b** zwischen der Spülgas-Zufuhröffnung **3** und der Biogas-Entnahmeöffnung **2** kann also je nach Betriebszustand in unterschiedlichen Richtungen und von einem unterschiedlichen Gas durchströmt werden, nämlich von Biogas oder Abgas als Spülgas.

Falls einer der Fermenter **1** mit einem Spülgas, beispielsweise dem Abgas des Biogas-Verbrauchers **10,** gespült wird, vermischt sich dieses Spülgas im Gasraum des Fermenters **1** mit dem dort entstehenden oder vorhandenen Biogas und bildet ein Schwachgas, welches über die Schwachgas-Entnahmeöffnung **17** und die die Schwachgas-Sammelleitung **4** dem Zwischenspeicher **6** für Schwachgas zugeführt wird oder einem der anderen Fermenter, was über die entsprechende Öffnungs- oder Schließzustände der diesen Weg festlegenden Ventile geregelt wird.

Wie in Figur **1** dargestellt, wird das Schwachgas aus dem Schwachgas-Zwischenspeicher **6** wahlweise, gegebenenfalls zeitversetzt, einem oder mehreren der Fermenter **1** zugeführt, was an unterschiedlichen Stellen geschehen kann:

Beim rechten Fermenter **1** wird das Schwachgas über eine Schwachgas-Versorgungsleitung **14** und eine Schwachgas-Zufuhröffnung **25,** die sich in der Stichleitung **9b** von der Biogas-Entnahmeöffnung **2** zur Biogas-Sammelleitung **9** befindet, stromaufwärts einer Messstelle **5** in der Stichleitung **9b** zugeführt, welches die Zusammensetzung des dadurch entstehenden Gasgemisches misst.

Am linken Fermenter **1** befindet sich diese Schwachgas-Zufuhröffnung **25** dagegen in der Stichleitung von der Abluft-Entnahmeöffnung **23** zur Abluft-Sammelleitung **11** mit der Folge, dass das hier zugeführte Schwachgas im Gasraum des Fermenters **1** einen langen Weg von der Abluft-Entnahmeöffnung **23** bis zur entfernten Frischluft-Zufuhröffnung **22** zurücklegen muss und sich in dieser Zeit mit dem darin befindlichen Biogas gut vermischen kann.

Ferner ist in Figur **1** ein Perkolat-Zwischenspeicher **15** eingezeichnet, in dem das in den einzelnen Fermentern **1** anfallende Perkolat zwischengespeichert wird, bevor es in den einzelnen Fermentern **1** auf der Oberseite der Biomasse **7** wieder ausgebracht wird. In diesen Zwischenspeicher **15** bildet sich ebenfalls Biogas, da das Perkolat aktive Gärstoffe enthält, weshalb der Perkolat-Zwischenspeicher **15** auch eine Biogas-Entnahmeöffnung **2** aufweist, die mit der Biogas-Sammelleitung **9** in Verbindung steht.

Von der Schwachgas-Versorgungsleitungen **14** kann das Schwachgas auch dem Perkolat -Zwischenspeicher **15** zugeführt werden,.

Die Schwachgas-Sammelleitung **4** verzweigt sich am Ende entweder zu dem Abluftkamin **19** oder Fackel **13,** wofür vorher an einer Messstelle **5** die Zusammensetzung des Gases ermittelt und danach die Verzweigung gesteuert wird.

Ferner besteht eine Verbindung zwischen der Biogas-Sammelleitung **9** und der Schwachgas-Sammelleitung **4** an deren Stromabwärtigen Ende, also in Strömungs-Richtung des Biogases nach dem letzten Fermenter **1,** um bei Bedarf auch das Biogas entweder dem Abluftkamin **19** oder der Fackel **13** zuführen zu können. Von diesem Verbindungspunkt existiert auch eine Verbindung zum Schwachgas-Zwischenspeicher **6,** um das Schwachgas dort zwischenspeichern zu können. Ebenso ist die Biogas-Entnahmeöffnung **2** des Perkolat -Zwischenspeichers **15** mit der Biogas- Sammelleitung **9** verbunden.

Welches Gas in welcher Leitung von wo nach wo strömt, wird durch die eingebauten Ventile **20** a, b geregelt und das in Gang setzen oder abschalten der notwendigen Gebläse **21a,b,** was von einer nicht dargestellten Steuerung, die mit allen Ventilen, Gebläsen und Messstellen **5** in Verbindung steht, geregelt wird.

Somit kann für jeden Fermenter **1** separat mit Hilfe der Steuerung festgestellt und gesteuert werden, welche Zusammensetzung das aus diesem Fermenter **1** stammende Gas oder Gasgemisch besitzt, und abhängig davon, in welche der Sammelleitungen **9,** oder **11/4** es eingeleitet werden soll.

Sofern im Normalbetrieb der Biogas-Anlage das Gasgemisch zwar Methan enthält, aber zu wenig, um vom Biogas-Verbraucher **10** verwertet werden zu können, wird ein solches Gasgemisch nicht entsorgt, sondern dem Schwachgas-Zwischenspeicher **6** über die Schwachgas-Sammelleitung **4** zugeführt und von dort aus - ggf. zeitversetzt - dem Biogas anderer Fermenter **1** zugesetzt, jedoch nur in so geringem Maß, dass, insbesondere gerade noch, das entstehende Gasgemisch dieser anderen Fermenter problemlos und damit dem Biogas-Verbraucher **10** zugeführt werden und dort verbrannt werden kann.

Die Biogas-Anlage gemäß **Figur 2** unterscheidet sich von derjenigen der Figur **1** lediglich dadurch, dass die Schwachgas-Versorgungsleitung **14,** insbesondere von dem Schwachgas-Zwischenspeicher, nur zu einem einzigen Einspeisepunkt **8** in der Biogas-Sammelleitung **9** geführt wird, und dieser Einspeisepunkt **8** zwischen der Mündung der letzten Stichleitung von einem Fermenter **1** und dem Anschluss an den Biogasverbraucher **10** liegt.

In **Figur 3** ist ein einzelner Fermenter **1** dargestellt, in dessen Oberseite sich die beiden Öffnungen, nämlich die Frischluft-Zufuhröffnung **22** mit dem davon nach oben wegführenden Frischluftstutzen **22** sowie die in der diagonal gegenüberliegenden Ecke angeordnete Abluft-Entnahmeöffnung **23** mit dem davon nach oben wegführenden Abluftstutzen **23** befinden.

Beide Stutzen enthalten beabstandet vom Fermenter **1** je ein Einsperrventil, und zwischen dem Sperrventil und dem Fermenter **1,** also der jeweiligen Mündung des Stutzens im Fermenter **1,** mündet je wenigstens eine Abzweigleitung oder Stichleitung.

Ferner ist zu erkennen, dass in der von der Abluft-Entnahmeöffnung **23** wegführenden Stichleitung zur Schwachgas-Sammelleitung **4** die Schwachgas-Versorgungsleitung **14** in einer Schwachgas-Zuführöffnung **25,** die gleichzeitig Schwachgas-Entnahmeöffnung **17** sein kann, mündet, um bei Bedarf Schwachgas über die Abluft-Entnahmeöffnung **23** in den Fermenter **1** einspeisen zu können.

An dem Frischluftstutzen **22** ist ferner eine Biogas-Entnahmeöffnung **2** vorhanden, die über eine Stichleitung **9a,** die der Überdruck-Sicherung **24** zugeführt wird, mit der nicht dargestellten Biogas-Sammelleitung **9** verbunden ist. In der Stichleitung **9a** mündet die Spülgas-Versorgungsleitung **16,** um hier bei Bedarf Spülgas, zum Beispiel das Abgas des Biogasverbrauchers, in den Fermenter **1** einzuspeisen. Hier oder über die Entnahmeöffnung **23** kann auch Schwachgas eingespeist werden.

### BEZUGSZEICHENLISTE

- **1**: Fermenter
- **2**: Biogas-Entnahmeöffnung
- **3**: Spülgas-Zufuhröffnung
- **4**: Schwachgas-Sammelleitung
- **5**: Messstelle
- **6**: Zwischenspeicher
- **7**: Biomasse
- **8**: Einspeisepunkt
- **9**: Biogas-Sammelleitung
- **9**a, b: Stichleitung
- **10**: Biogas-Verbraucher
- **11**: Abluft-Sammelleitung
- **12**: Abfackel-Sammelleitung
- **13**: Fackel
- **14**: Schwachgas-Versorgungsleitung
- **15**: Perkolat-Zwischenspeicher
- **16**: Spülgas-Versorgungsleitung
- **17**: Schwachgas-Entnahmeöffnung
- **18 19**: Abluft-Kamin
- **20**a,b: Ventil
- **21**a: Gebläse
- **22**: Frischluft-Zufuhröffnung, Frischluft stutzen
- **23**: Abluft-Entnahmeöffnung, Abluftstutzen
- **24**: Überdruck Sicherung
- **25**: Schwachgas-Zufuhröffnung

## Patentansprüche

1. **Biogasanlage** zur Erzeugung und Verwertung von Biogas aus nichtpumpbarer Biomasse **(7),** mit
- mehreren gas- und flüssigkeitsdicht verschließbaren Fermentern **(1)** für die Biomasse **(7),**
- einer Biogas-Entnahmeöffnung **(2),** einer Schwachgas-Entnahmeöffnung **(17)** und einer Abluft-Entnahmeöffnung **(23)** in jedem Fermenter **(1),**
- einer Spülgas-Zufuhröffnung **(3)** in jedem Fermenter **(1),**
- einer Schwachgas-Sammelleitung **(4),** die mit jedem Fermenter **(1)** verbunden ist,
- einem Biogas-Verbraucher **(10**
**gekennzeichnet durch**
- eine Dosiersteuerung **(5),** die für eine solche Zudosierung von Schwachgas in das Biogas veranlasst, dass das Biogas für den Biogas-Verbraucher **(10)** nutzbar ist.

2. Biogasanlage nach Anspruch **1,**
**dadurch gekennzeichnet, dass**
die Biogasanlage einen Zwischenspeicher **(6)** für Schwachgas umfasst.

3. Biogasanlage nach einem der vorhergehenden Ansprüche,
**dadurch gekennzeichnet, dass**
jeder Fermenter **(1)** eine Wählsteuerung für die Zuführung des aus ihm entnommenen Gas-Gemisches wahlweise zur Schwachgas-Sammelleitung **(4),** zur Biogas-Sammelleitung **(9),** zur Abluft-Sammelleitung **(11)** oder zur Abfackel-Sammelleitung **(12)** aufweist, wobei insbesondere die Schwachgas-Sammelleitung **(4)** streckenweise mit der Abfackel-Sammelleitung **(12)** identisch sein kann.
(Dezentrale Zudosierung)

4. Biogasanlage nach einem der vorhergehenden Ansprüche,
**dadurch gekennzeichnet, dass**
jeder Fermenter **(1)** separat mit einer Schwachgas-Versorgungsleitung **(14)** verbunden ist.

5. Biogasanlage nach Anspruch **4,**
**dadurch gekennzeichnet, dass**
- entweder die Schwachgas-Versorgungsleitung **(14)** nahe der Biogas - Entnahmeöffnung **(2)** jedes Fermenters **(1),** insbesondere auf der Außenseite der im Fermenter (1) vorhandenen Biogas-Entnahmeöffnung **(2)** oder Frischluft-Zuführöffnung (22), mündet,
- oder die Schwachgas-Versorgungsleitung **(14)** nahe der Abluft-Entnahmeöffnung **(23)** jedes Fermenters (1), insbesondere auf der Außenseite der im Fermenter (1) vorhandenen Abluft-Entnahmeöffnung **(23),** mündet,
- oder die Schwachgas-Versorgungsleitung **(14)** im Fermenter so weit wie möglich entfernt von der Biogas-Entnahmeöffnung **(2)** mündet, innerhalb derselben Biogasanlage jedoch nur eine der Möglichkeiten eingesetzt wird.

6. Biogasanlage nach einem der vorhergehenden Ansprüche,
**dadurch gekennzeichnet, dass**
jeder Fermenter **(1)** separat, insbesondere die Biogas-Entnahmeöffnung **(2)** jedes Fermenters **(1),** eine separate Dosiersteuerung **(5)** für die Zudosierung von Schwachgas in das Biogas dieses Fermenters **(1)** aufweist.
(Zentrale Zudosierung)

7. Biogasanlage nach einem der vorhergehenden Ansprüche,
**dadurch gekennzeichnet, dass**
in der Sammelleitung **(9)** für Biogas stromabwärts der letzten Stichleitung **(9b)** von den einzelnen Fermentern **(1),** insbesondere unmittelbar vor dem Biogas-Verbraucher **(10)** oder einer Biogas-Aufbereitungsvorrichtung, eine Dosiersteuerung **(5)** für die Zudosierung von Schwachgas in das Biogas angeordnet ist.

8. **Verfahren** zur Erzeugung und Verwertung von Biogas aus nichtpumpbarer Biomasse **(7),** mit
- mehreren gas- und flüssigkeitsdicht verschließbaren Fermentern **(1)** für die Biomasse **(7),**
- einer Biogas-Entnahmeöffnung **(2),** einer Schwachgas-Entnahmeöffnung **(17)** und einer Abluft-Entnahmeöffnung **(23)** in jedem Fermenter **(1),**
- einer Spülgas-Zufuhröffnung **(3)** in jedem Fermenter **(1),**
- einem Biogas-Verbraucher (**10**,
**dadurch gekennzeichnet, dass**
Schwachgas dem Biogas in einem so geringen Umfang zugesetzt wird, dass die sich einstellende Mischung aus Schwachgas und Biogas noch nutzbar, insbesondere für den angeschlossenen Biogas-Verbraucher **(10)** nutzbar, ist und diesem insbesondere zugeführt wird.

9. Verfahren nach Anspruch **8,**
**dadurch gekennzeichnet, dass**
das in einem Fermenter **(1)** entstehende Schwachgas mit oder ohne Zwischenspeicherung in einem Schwachgas-Speicher **(6)** einem oder mehreren der anderen Fermenter **(1),** dem Perkolatzwischenspeicher **(15)** oder der Biogas-Sammelleitung **(9)** zugeführt wird.
(Dezentrale Zudosierung)

10. Verfahren nach Anspruch **8** oder **9,**
**dadurch gekennzeichnet, dass**
das Schwachgas dem Biogas gesteuert an jedem einzelnen Fermenter **(1)** zudosiert wird.

11. Verfahren nach Anspruch **10,**
**dadurch gekennzeichnet, dass**
- entweder das Schwachgas dem Biogas nahe der Biogas-Entnahmeöffnung **(2)** jedes Fermenters **(1),** insbesondere auf der Außenseite der im Fermenter **(1)** vorhandenen Biogas-Entnahmeöffnung **(2)** zudosiert wird
- oder das Schwachgas im Fermenter oder dem Perkolatzwischenspeicher **(15)** so weit wie möglich entfernt von der Biogas-Entnahmeöffnung (2) zudosiert wird.

12. Verfahren nach einem der vorhergehenden Verfahrensansprüche,
**dadurch gekennzeichnet, dass**
die Zusammensetzung des Gas-Gemisches an der Gas-Entnahmeöffnung **(2, 17)** jedes einzelnen Fermenters **(1)** oder des Perkolatzwischenspeichers **(15)** ermittelt wird.
(Zentrale Zudosierung)

13. Verfahren nach einem der vorhergehenden Verfahrensansprüche,
**dadurch gekennzeichnet, dass**
das Schwachgas dem Biogas in der Sammelleitung **(9)** für Biogas stromabwärts der letzten Stichleitung **(9** b) von den einzelnen Fermentern **(1),** insbesondere unmittelbar vor dem Biogas-Verbraucher **(10)** oder einer Biogas-Aufbereitungsvorrichtung, zugeführt wird.

14. Verfahren nach einem der vorhergehenden Verfahrensansprüche,
**dadurch gekennzeichnet, dass**
die Zusammensetzung des Gas-Gemisches in der Sammelleitung **(9)** für Biogas stromabwärts der letzten Stichleitung **(9** b) von den einzelnen Fermentern **(1),** ermittelt wird.

15. Verfahren nach einem der vorhergehenden Verfahrensansprüche,
**dadurch gekennzeichnet, dass**
die Zuführung des aus einem Fermenter **(1)** entnommenen Gas-Gemisches wahlweise zur Schwachgas-Sammelleitung **(4),** zur Biogas-Sammelleitung **(9),** zur Abluft-Sammelleitung **(11)** oder zur Abfackel-Sammelleitung **(12)** für jeden Fermenter **(1)** individuell gesteuert wird.
